# EUROPÄISCHE PATENTANMELDUNG

(11) **EP 3 939 730 A1**
(43) Veröffentlichungstag der Anmeldung: **19.01.2022**
(21) Anmeldenummer: 20185691.1
(22) Anmeldetag: 14.07.2020
(51) Int. Cl.: B23K 1/00, A61N 1/375, B23K 20/00, B23K 20/16, B23K 20/22, B23K 101/36, B23K 101/38

(54) **VERFAHREN ZUM FÜGEN EINER ERSTEN UND EINER ZWEITEN KOMPONENTE EINER ELEKTRISCHEN DURCHFÜHRUNG UNTER VERWENDUNG EINES REAKTIVEN MULTISCHICHTSYSTEMS**

(71) Anmelder: BIOTRONIK SE & Co. KG, 12359 Berlin (DE)
(72) Erfinder: KRONMUELLER, Daniel, 90409 Nuernberg (DE)
(74) Vertreter: Biotronik Corporate Services SE

(57) **Zusammenfassung**

Verfahren zum Fügen einer ersten (20, 13) und einer zweiten Komponente (3, 25, 70) einer elektrischen Durchführung (11) für ein medizinisches Gerät (1), aufweisend die Schritte: Bereitstellen der ersten Komponente (20, 13), der zweiten Komponente (3, 25, 70) und eines reaktiven Multischichtsystems (40) aufweisend alternierend angeordnete erste und zweite Materiallagen (41, 42), wobei die ersten Materiallagen (41) einen ersten Stoff (A) aufweisen und wobei die zweiten Materiallagen (42) einen zweiten Stoff (B) aufweisen, und Anregen des reaktiven Multischichtsystems (40) mit einer Aktivierungsenergie, so dass die beiden Stoffe (A, B) unter Freisetzung von Wärmeenergie exotherm miteinander reagieren, wobei die Wärmeenergie zum Fügen der ersten und der zweiten Komponente (20, 13; 3, 25, 70) verwendet wird.

## Beschreibung

Die Erfindung betrifft eine elektrische Durchführung für ein medizinisches Gerät, insbesondere für ein implantierbares medizinisches Gerät, z.B. in Form eines Herzschrittmachers, eines Kardioverter-Defibrillators (ICD) oder eines Neurostimulators.

Das Herstellen von medizinischen Durchführungen ist ausführlich in der Literatur beschrieben (z. B. EP 2 371 418 A2). Hierbei werden die Komponenten mittels eines biokompatiblen Lotes gefügt. Zum erfolgreichen Fügen müssen in der Regel alle Komponenten annähernd auf die Schmelztemperatur des Lotes gebracht werden. Hierzu sind verschiedene Verfahren bekannt, z.B. das Ofenlöten, Vakuumlöten.

Des Weiteren ist bekannt, dass es energetisch vorteilhaft ist, nur das Lot und die unmittelbar angrenzenden Bauteile für den Fügevorgang zu einer Durchführung zu erwärmen (US 9,478,959 B2). Dies kann zum Beispiel mit einem Laserstrahl erfolgen, welcher in der Lage ist selektiv das Lot und angrenzende Bereiche zu erwärmen. Hierbei wird durch den Laser ein Gradientenfeld im Temperaturfeld des Bauteils erzeugt, welcher im Bereich der Energieeinkopplung seine maximale Temperatur hat.

Wird die Durchführung, wie in der Literatur beschrieben, komplett durchwärmt, sind enorme Energiemengen erforderlich, um zu erwärmen. Dies liegt daran, dass ausreichend Energie durch die Fertigungshilfsmittel und Komponenten fließen muss, bis die Schmelztemperatur am Lot überschritten wurde. Weiterhin benötigt die Erwärmung je nach Wärmekapazität der Komponenten ausreichend Zeit bis sich ein homogenes Temperaturfeld auf den Bauteilen eingestellt hat, und das Lot nicht nur partiell sondern homogen aufschmilzt. Hierfür ist es erforderlich, dem Bauteil während der Durchwärmung ausreichend Zeit zur Relaxation bzw. Homogenisierung des Temperaturgradienten zu lassen.

Ebenso muss die Wärme von den Bauteilen abgeführt werden, damit die Fügeverbindung erfolgreich hergestellt werden kann. Erst dann kann die Durchführung weiter prozessiert und verarbeitet werden. Hierbei treten die eben beschrieben Probleme wieder in Erscheinung. Dies muss möglichst homogen und gleichmäßig passieren, um Spannungen in den Bauteilen zu verhindern. Ferner muss das Lot zum Erstarren gebracht werden, bevor sich in der Schmelze zu viele Legierungsbestandteile aus anliegenden Komponenten gelöst haben.

Ist es nicht möglich, das Bauteil komplett zu Durchwärmen (z.B. wegen temperaturempflicher Elektronik im Inneren des medizinischen Gerätes), so werden nur einzelne Komponenten des medizinischen Gerätes selektiv erwärmt (vgl. WO 2014/190256 A3). Durch gezieltes Erwärmen eines Bauteils verteilt sich die Wärmeenergie mittels Strahlung, Konvektion und Wärmeleitung auf die anliegenden Komponenten, wodurch sich ein Wärmegleichgewicht auf der Baugruppe einstellt. Die Wärmeenergie kann durch Photonen (Laserschweißen) oder durch Elektronen (Elektronenstrahlschweißen) in das Werkstück eingebracht werden. Beim selektiven Erwärmen des Bauteils (z.B. mittels Laser oder Elektronenstrahl) muss die Fügestelle kontinuierlich hintereinander abgerastert werden, wodurch sich die Wärme ebenfalls allmählich in die angrenzenden Bauteile ausbreitet und dabei deutlich den Fügebereich verlässt.

Hiervon ausgehend liegt der Erfindung die Aufgabe zugrunde, ein verbessertes Verfahren zum Fügen zumindest einer ersten und einer zweiten Komponente einer elektrischen Durchführung für ein medizinisches Gerät anzugeben bzw. eine verbesserte elektrische Durchführung bereitzustellen.

Diese Aufgabe wird durch ein Verfahren mit den Merkmalen des Anspruchs 1 sowie durch eine elektrische Durchführung mit den Merkmalen des Anspruchs 15 gelöst.

Ein weiterer Erfindungsaspekt betrifft ein medizinisches Gerät mit einer erfindungsgemäßen elektrischen Durchführung.

Vorteilhafte Ausgestaltungen der Erfindungsaspekte sind in den entsprechenden Unteransprüchen angeben und werden nachfolgend beschrieben.

Gemäß Anspruch 1 wird ein Verfahren zum Fügen einer ersten und einer zweiten Komponente einer elektrischen Durchführung für ein medizinisches Gerät offenbart, wobei das Verfahren die Schritte aufweist:
- Bereitstellen der ersten Komponente, der zweiten Komponente und eines reaktiven Multischichtsystems aufweisend alternierend angeordnete erste und zweite Materiallagen, wobei die ersten Materiallagen einen ersten Stoff aufweisen und wobei die zweiten Materiallagen einen zweiten Stoff aufweisen; und
- Anregen des reaktiven Multischichtsystems mit einer Aktivierungsenergie, so dass die beiden Stoffe unter Freisetzung von Wärmeenergie exotherm miteinander reagieren, wobei die Wärmeenergie zum Fügen der ersten und der zweiten Komponente verwendet wird.

Bei dem medizinischen Gerät handelt es sich insbesondere um ein implantierbares medizinisches Gerät, z.B. einen Herzschrittmacher, einen Kardioverter-Defibrillator oder einen Neurostimulator.

Eine derartige elektrische Durchführung kann zumindest einen elektrisch leitfähigen Stift (oder mehrere solche Stifte) aufweisen, der sich durch einen Isolator der elektrischen Durchführung hindurcherstrecken kann, wobei der Isolator aus einem Glas oder einer Keramik gebildet sein kann. Der mindestens eine Stift kann mit dem Isolator über eine Fügeverbindung, beispielsweise eine Lötverbindung, stoffschlüssig, vorzugsweise hermetisch, verbunden sein (z.B. wenn der Isolator aus einer Keramik oder einem Glas gebildet ist).

Der Isolator kann weiterhin mit einem umlaufenden Flansch der elektrischen Durchführung stoffschlüssig verbunden sein, wobei der Flansch wiederum mit einem Gehäuse des medizinischen Geräts stoffschlüssig, vorzugsweise hermetisch, verbunden sein kann. Mindestens eine der beiden stoffschlüssigen, vorzugsweise hermetischen, Verbindungen können z.B. mit dem erfindungsgemäßen Verfahren hergestellt werden.

Der mindestens eine Stift der elektrischen Durchführung ist im Innenraum des Gehäuses insbesondere mit einer Leiterbahn einer Leiterplatte einer elektronischen Schaltung des medizinischen Geräts elektrisch leitfähig verbunden. Hierzu können typischerweise verschiedene Fügeverfahren eingesetzt werden, zum Beispiel kann die Verbindung mittels Weichlötverbindung, mittels eine Bondprozesses oder einer Schweißung hergestellt werden. Darüber hinaus sind auch andere Fertigungsverfahren wie zum Beispiel Sintern und elektrisch leitfähig Kleben vorstellbar.

Die vorliegende Erfindung erlaubt damit mit Vorteil die Fügung der Komponenten bei möglichst geringer Temperatur da die notwendige Wärmeenergie direkt an der Fügestelle bereitgestellt werden kann, so dass mechanische Spannungen nicht in das Bauteil eingeprägt werden. Weiterhin kann die Fügung stattfinden, ohne dass die Bauteile vollständig durchwärmt werden müssen, was ebenfalls von Vorteil ist. Hierbei ist es idealerweise sogar möglich, eine Fügung zu erzielen, ohne dass die beteiligten Stoffe vom festen in den flüssigen Zustand wechseln müssen. Weiterhin kann eine solche Fügung auch hergestellt werden, ohne dass alle Bereiche einsehbar sind (z.B. durch Einbringen einer Aktivierungsenergie in Form einer Strahlung, z.B. Laserlicht über eine geeignete Optik).

Gemäß einer Ausführungsform des Verfahrens ist vorgesehen, dass eine stoffschlüssige Verbindung zwischen der ersten und der zweiten Komponente durch eine Mischphase des ersten und des zweiten Stoffes gebildet wird. Hierbei werden also mit anderen Worten die beiden Komponenten mit Hilfe des reaktiven Multischichtsystems durch Diffusion der beiden Stoffe stoffschlüssig miteinander gefügt.

Weiterhin ist gemäß einer Ausführungsform des Verfahrens vorgesehen, dass das reaktive Multischichtsystem vor dem Anregen zwischen der ersten und der zweiten Komponente angeordnet wird.

Weiterhin ist gemäß einer Ausführungsform des Verfahrens vorgesehen, dass das reaktive Multischichtsystem vor dem Anregen bereits auf die erste Komponente oder auf die zweite Komponente aufgebracht ist.

Weiterhin ist gemäß einer Ausführungsform des Verfahrens vorgesehen, dass auf dem reaktiven Multischichtsystem ein Lotmaterial angeordnet ist, insbesondere ein eutektisches Lotmaterial, wobei das Lotmaterial durch die Wärmenergie aufgeschmolzen wird, so dass eine Lötverbindung zwischen der ersten und der zweiten Komponente ausgebildet wird.

Insbesondere kann mit Hilfe des reaktiven Multischichtsystems ein Wärmeeintrag in die Fügepartner reduziert werden, da das reaktive Multischichtsystem so angeordnet werden kann, dass es gezielt nur die Fügestelle und das Lotmaterial über den Schmelzpunkt des Lotmaterials erwärmt.

Gemäß einer Ausführungsform des Verfahrens ist vorgesehen, dass die Aktivierungsenergie zur Anregung des reaktiven Multischichtsystems durch Bestrahlen des Multischichtsystems mit Laserlicht bereitgestellt wird. Alternativ hierzu kann vorgesehen sein, dass die Aktivierungsenergie zur Anregung des reaktiven Multischichtsystems durch Ausüben eines mechanischen Drucks auf das reaktive Multischichtsystem bereitgestellt wird. Es ist auch denkbar die beiden Varianten zu kombinieren, d.h., Einstrahlen von Laserlicht und Ausüben eines mechanischen Drucks zur Bereitstellung der notwendigen Aktivierungsenergie.

Weiterhin ist gemäß einer Ausführungsform des Verfahrens vorgesehen, dass die erste Komponente ein Flansch der elektrischen Durchführung ist, wobei der Flansch aus einem Metall oder einer Metalllegierung gebildet sein kann, insbesondere aus Titan, einer Titanlegierung, Edelstahl der Qualität 316L, Nitinol oder Niob.

Dabei kann es sich gemäß einer Ausführungsform der Erfindung bei der zweiten Komponente um ein Gehäuse des medizinischen Gerätes handeln. Das Gehäuse kann z.B. aus Titan, einer Titanlegierung, Edelstahl der Qualität 316L, Nitinol oder Niob gebildet sein.

Gemäß einer alternativen Ausführungsform des Verfahrens ist vorgesehen, dass die zweite Komponente ein Isolator der elektrischen Durchführung ist, wobei sich zumindest ein elektrisch leitfähiger Stift (oder mehrere solche Stifte) durch den Isolator hindurch erstreckt. Der Isolator kann aus einem Glas oder einer Keramik gebildet sein, wobei der mindestens eine Stift über eine Lötverbindung stoffschlüssig mit dem Isolator verbunden sein kann (z.B. wenn es sich bei dem Isolator um einen keramischen Isolator handelt). Der mindestens eine Stift kann eines der folgenden Metalle aufweisen: Platin, Platin/Iridium, Niobium, Titan, Tantal, Palladium, Kupfer, Nickel, Gold, Silber, Rhodium, Ruthenium, Osmium, Wolfram. Dabei kann der Stift der Durchführung mehrere Metalle enthalten. Typischerweise enthält der Stift nahe der Platine bzw. Elektronik typische Metalle und Legierungen der Leiterplattentechnik (z.B. Kupfer, Nickel, Silber, Palladium) und dem Körper zu gewandten Seite biokompatible Materialien (z.B. Niob, Titan, Platin, Iridium).

Falls Laserlicht zur Anregung verwendet wird, kann die erste oder die zweite Komponente eine Öffnung aufweisen, über die das Laserlicht zum reaktiven Multischichtsystem geführt wird (z.B. mittels einer geeigneten Optik, insbesondere faserbasierte Optik).

Weiterhin ist gemäß einer Ausführungsform des Verfahrens vorgesehen, dass die erste Komponente ein elektrisch leitfähiger Stift der elektrischen Durchführung ist, der sich insbesondere durch einen bzw. den Isolator der elektrischen Durchführung hindurch erstreckt.

Weiterhin kann es sich gemäß einer Ausführungsform des Verfahrens bei der zweite Komponente um eine Leiterplatte einer elektrischen Schaltung des medizinischen Geräts handeln. Hierbei kann zum Beispiel die Wärmeenergie des reaktiven Multischichtsystems dazu genutzt werden, die Leiterplatte und Durchführung miteinander zu fügen (z.B. mittels Weichlot).

Gemäß einer bevorzugten Ausführungsform des Verfahrens ist diesbezüglich vorgesehen, dass das reaktive Multischichtsystem insbesondere vor dem Anregen mit der Aktivierungsenergie auf einem Endabschnitt des mindestens einen Stiftes aufgebracht ist. Falls Laserlicht zur Anregung verwendet wird, kann z.B. das Gehäuse eine Öffnung aufweisen, über die das Laserlicht zum reaktiven Multischichtsystem geführt wird (z.B. mittels einer geeigneten Optik, insbesondere faserbasierte Optik).

Vorliegend wurde die Erfindung jeweils anhand von zwei Komponenten der elektrischen Durchführung beschrieben, die mittels des erfindungsgemäßen Verfahrens miteinander stoffschlüssig verbunden werden. Diese einzelnen Ausführungsformen können beliebig miteinander kombiniert werden, d.h., eine oder mehrere der folgenden Komponentenpaare können nach den hierin beschriebenen Ausführungsformen des erfindungsgemäßen Verfahrens miteinander stoffschlüssig verbunden werden: der Flansch und das Gehäuse (des medizinischen Gerätes), der Flansch und der Isolator, der mindestens eine Stift und die Leiterplatte.

Gemäß einer bevorzugten Ausführungsform des Verfahrens ist vorgesehen, dass der erste Stoff der ersten Materiallagen und der zweite Stoff der zweiten Materiallagen aus der Gruppe der folgenden Kombinationen ausgewählt sind, wobei jeweils zunächst der erste Stoff und dann der zweite Stoff genannt ist:
- Al (Aluminium) und Ni (Nickel),
- Al (Aluminium) und Pt (Platin),
- Al (Aluminium) und Zr (Zirconium),
- Nb (Niob) und Si (Silizium),
- Ni (Nickel) und Ti (Titan),
- Si (Silizium) und Ti (Titan),,
- Pt (Platin) und Al (Aluminium),
- Ti (Titan) und Si (Silizium),
- Ti (Titan) und C (Kohlenstoff).

Diese Stoffkombinationen führen nach initialer Zündung mittels der Aktivierungsenergie zu den nachfolgend aufgelisteten exothermen Reaktionen bzw. Mischphasen. Ebenfalls angegeben sind die jeweils freigesetzte Wärmeenergie sowie die maximale Reaktionstemperatur. Die freigesetzte Wärme wird dazu genutzt, die Werkstoffe mittels Diffusion zu bonden bzw. ein Lotmaterial zu schmelzen.

| Erster Stoff | Zweiter Stoff | Reaktion | H_{f}⁰[kJ/mol] | Tmax[°C] |
|---|---|---|---|---|
| Al | Ni | Al+Ni | -58,3 | 2089 |
| Al | Pt | Al+Pt | -94,9 | 3106 |
| Al | Zr | 2Al+Zr | -52,1 | 1650 |
| Nb | Si | 5Nb+3Si | -52,4 | 1565 |
| Ni | Ti | Ni+Ti | -33,1 | 1310 |
| Si | Ti | 3 Si+Ti | -73,8 | 2275 |
| Pt | Al | Pt+Al | -100,2 | 3379 |
| Ti | Si | Ti+Si | -72,6 | 1640 |
| Ti | C | Ti+C | -92,9 | 3371 |

Weiterhin ist gemäß einer Ausführungsform des Verfahrens vorgesehen, dass das reaktive Multischichtsystem eine Gesamtanzahl von Materiallagen aufweist (erste und zweite Materiallagen zusammengenommen), die im Bereich von 2 Materiallagen bis 10.000 Materiallagen liegt, vorzugsweise im Bereich von 10 Materiallagen bis 70 Materiallagen.

Weiterhin ist gemäß einer Ausführungsform der Erfindung vorgesehen, dass die jeweilige erste Materiallage eine Schichtdicke im Bereich von 1 nm bis 2000 nm aufweist, vorzugsweise im Bereich von 20 nm bis 500 nm. Gemäß einer Ausführungsform der Erfindung weist weiterhin die jeweilige zweite Materiallage eine Schichtdicke im Bereich von 1 nm bis 2000 nm auf, vorzugsweise im Bereich von 20 nm bis 500 nm.

Ein weiterer Aspekt der vorliegenden Erfindung betrifft eine elektrische Durchführung für ein medizinisches Gerät, insbesondere für ein implantierbares medizinisches Gerät, wobei die elektrische Durchführung eine erste und eine zweite Komponente aufweist, die mittels des Verfahrens nach einem der vorhergehenden Ansprüche gefügt sind, d.h. stoffschlüssig, insbesondere hermetisch, miteinander verbunden sind.

Insbesondere weist die erfindungsgemäße elektrische Durchführung zumindest einen elektrisch leitfähigen Stift auf, der sich durch einen Isolator der elektrischen Durchführung hindurcherstreckt, wobei der Isolator aus einem Glas oder einer Keramik gebildet sein kann, und wobei der Stift mit dem Isolator über eine Lötverbindung stoffschlüssig, insbesondere hermetisch, verbunden sein kann (z.B. wenn der Isolator aus einer Keramik gebildet ist).

Der Isolator ist bevorzugt mit einem umlaufenden Flansch der elektrischen Durchführung stoffschlüssig, insbesondere hermetisch, verbunden (z.B. mittels des erfindungsgemäßen Verfahrens), wobei der Flansch wiederum mit einem Gehäuse des medizinischen Geräts stoffschlüssig, insbesondere hermetisch, verbunden ist (z.B. mittels des erfindungsgemäßen Verfahrens).

Der mindestens eine Stift der elektrischen Durchführung ist im Innenraum des Gehäuses des medizinischen Geräts insbesondere mit einer Leiterbahn einer Leiterplatte einer elektronischen Schaltung des medizinischen Geräts elektrisch leitfähig verbunden, insbesondere über eine Lötverbindung (z.B. mittels des erfindungsgemäßen Verfahrens).

Bei der ersten und der zweiten Komponente kann es sich also um eines der folgenden Komponentenpaare handeln (siehe auch oben): der Flansch (erste Komponente) und das Gehäuse (zweite Komponente), der Flansch (erste Komponente) und der Isolator (zweite Komponente), der mindestens eine Stift (erste Komponente) und die Leiterplatte (zweite Komponente). Diese einzelnen Ausführungsformen können wiederum auch beliebig miteinander kombiniert werden, d.h. mehr als ein Komponentenpaar der Durchführung können mittels des erfindungsgemäßen Verfahrens gefügt werden.

Ein weiterer Aspekt der vorliegenden Erfindung betrifft ein medizinisches Gerät, insbesondere implantierbares medizinisches Gerät (z.B. einen Herzschrittmacher, einen Kardioverter-Defibrillator oder einen Neurostimulator), das eine erfindungsgemäße elektrische Durchführung aufweist oder das zumindest eine erste und eine zweite Komponente aufweist, die mit dem erfindungsgemäßen Verfahren gefügt sind.

Im Folgenden sollen Ausführungsformen der Erfindung sowie weitere Merkmale und Vorteile der Erfindung anhand der Figuren erläutert werden. Es zeigen:
- Fig. 1: eine schematische Ansicht einer Ausführungsform einer erfindungsgemäßen elektrischen Durchführung bzw. eines erfindungsgemäßen implantierbaren medizinischen Geräts (z.B. Herzschrittmacher),
- Fig. 2: eine Ausführungsform eines erfindungsgemäßen Verfahrens bzw. einer erfindungsgemäßen elektrischen Durchführung, wobei hier das reaktive Multischichtsystem auf eine Außenseite eines Flansches aufgebracht ist, die später dem Innenraum des Gehäuses des medizinischen Gerätes abgewandt ist, wobei das Multischichtsystem zur stoffschlüssigen Verbindung des Flansches mit einem Gehäuse des Gerätes dienen kann,
- Fig. 3: zeigt eine weitere Ausführungsform eines erfindungsgemäßen Verfahrens bzw. einer erfindungsgemäßen elektrischen Durchführung, wobei ein Flansch der Durchführung und ein Gehäuse des Geräts mittels eines reaktiven Multischichtsystems und zusätzlichem Lotmaterial gefügt werden,
- Fig. 4: zeigt eine weitere Ausführungsform eines erfindungsgemäßen Verfahrens bzw. einer erfindungsgemäßen elektrischen Durchführung, wobei ein Flansch der Durchführung und ein Isolator der Durchführung mittels eines reaktiven Multischichtsystems und optionalem Lotmaterial gefügt werden,
- Fig. 5: zeigt eine weitere Ausführungsform eines erfindungsgemäßen Verfahrens bzw. einer erfindungsgemäßen elektrischen Durchführung, wobei ein Flansch der Durchführung und ein Gehäuse des Geräts mittels eines reaktiven Multischichtsystems gefügt werden,
- Fig. 6: zeigt eine weitere Ausführungsform eines erfindungsgemäßen Verfahrens bzw. einer erfindungsgemäßen elektrischen Durchführung, wobei ein Flansch der Durchführung und zwei (oder mehrere) Isolatoren der Durchführung mittels reaktiven Multischichtsystemen gefügt werden, wobei eine zusätzliche Kunststoffdichtung zum Abdichten der Isolatoren verwendet wird,
- Fig. 7: zeigt eine weitere Ausführungsform eines erfindungsgemäßen Verfahrens bzw. einer erfindungsgemäßen elektrischen Durchführung, wobei ein Flansch der Durchführung und ein Gehäuse des Geräts mittels eines reaktiven Multischichtsystems und zusätzlichem Lotmaterial gefügt werden,
- Fig. 8: zeigt eine weitere Ausführungsform eines erfindungsgemäßen Verfahrens bzw. einer erfindungsgemäßen elektrischen Durchführung, wobei hier ein Stift der Durchführung und ein Kontakt einer Leiterplatte des Geräts mittels eines reaktiven Multischichtsystems gefügt werden; und
- Fig. 9: eine schematische Darstellung eines reaktiven Multischichtsystems, wie es bei den einzelnen Ausführungsformen verwendet werden kann, wobei das Multischichtsystem alternierend angeordnete Materiallagen mit einem ersten Stoff und einem zweiten Stoff aufweist, die nach Aktivierung exotherm miteinander reagieren und die zum Fügen verwendete Wärmeenergie bereitstellen.
- Fig. 10: zeigt eine weitere Ausführungsform eines erfindungsgemäßen Verfahrens bzw. einer erfindungsgemäßen elektrischen Durchführung, wobei ein Stift mittels eines reaktiven Multischichtsystems verlängert wurde, indem ein zweiter Stift angefügt wurde,

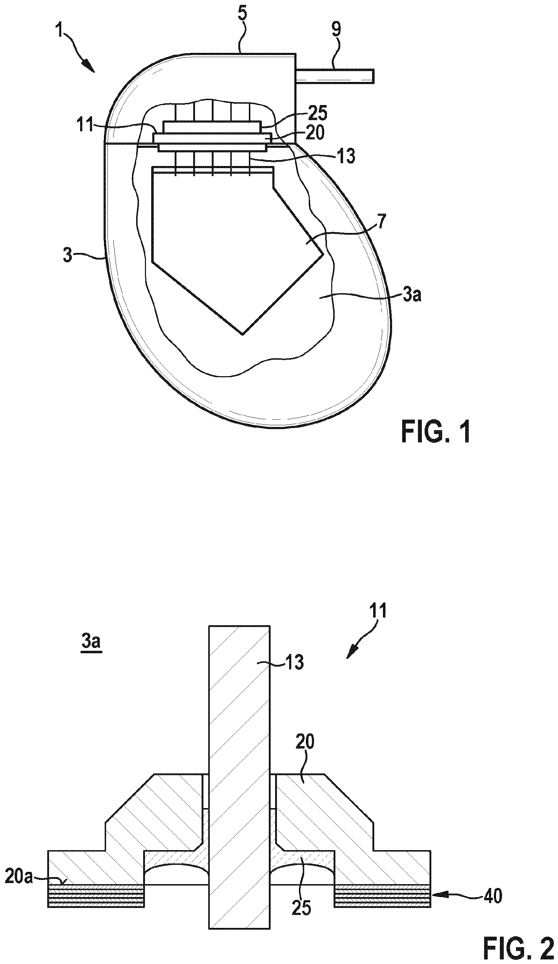

Fig. 1 zeigt eine schematische Darstellung einer erfindungsgemäßen elektrischen Durchführung 11 (auch "feedthrough" genannt). Eine solche Durchführung 11 stellt eine Anordnung von Komponenten bzw. eine Baugruppe dar, die es ermöglicht einen elektrische Verbindung über elektrisch leitende Stifte 13 zu schaffen, über die eine elektrische Schaltung 7 in einem Innenraum 3a eines hermetisch abgedichteten Gehäuses 3 eines insbesondere implantierbaren medizinischen Geräts 1 mit zumindest einer Komponente des Geräts 1 verbindbar ist, z.B. eine Buchse, die außerhalb des Gehäuses 3 angeordnet ist, z.B. in einem mit dem Gehäuse verbundenen Header 5. Über einen solchen Header 5 bzw. die Buchse kann z.B. eine Elektrodenleitung 9 an die Schaltung 7 angeschlossen werden. Bei dem implantierbaren medizinischen Gerät 1 kann es sich z.B. um einen Herzschrittmacher, einen Kardioverter-Defibrillator oder um einen Neurostimulator handeln. Die Erfindung ist jedoch auch elektrische Durchführungen 11 von anderen Geräten anwendbar.

Der mindestens eine elektrisch leitfähige Stift 13 erstreckt sich durch einen Isolator 25 hindurch, wobei der Isolator 25 z.B. aus einem Glas oder einer Keramik gebildet sein kann. Der mindestens eine Stift 13 kann mit dem Isolator 25 über eine Lötverbindung stoffschlüssig verbunden sein (z.B. wenn der Isolator 25 aus einer Keramik oder einem Glas gebildet ist). Der mindestens eine Stift 13 kann aus den folgenden Materialien gebildet sein bzw. kann eines der folgenden Materialien aufweisen: Platin, Platin/Iridium, Niobium, Titan, Tantal, Palladium, Kupfer, Nickel, Gold, Silber, Rhodium, Ruthenium, Osmium und/oder Wolfram.

Der Isolator 25 ist mit einem umlaufenden Flansch 20 der elektrischen Durchführung 11 stoffschlüssig, insbesondere hermetisch, verbunden, wobei der Flansch 20 wiederum mit dem Gehäuse 3 des medizinischen Geräts 1 stoffschlüssig, insbesondere hermetisch, verbunden ist. Diese beiden stoffschlüssigen, insbesondere hermetischen, Verbindungen können z.B. mit dem erfindungsgemäßen Verfahren hergestellt werden, das weiter unten anhand einzelner Ausführungsformen detailliert beschrieben werden wird.

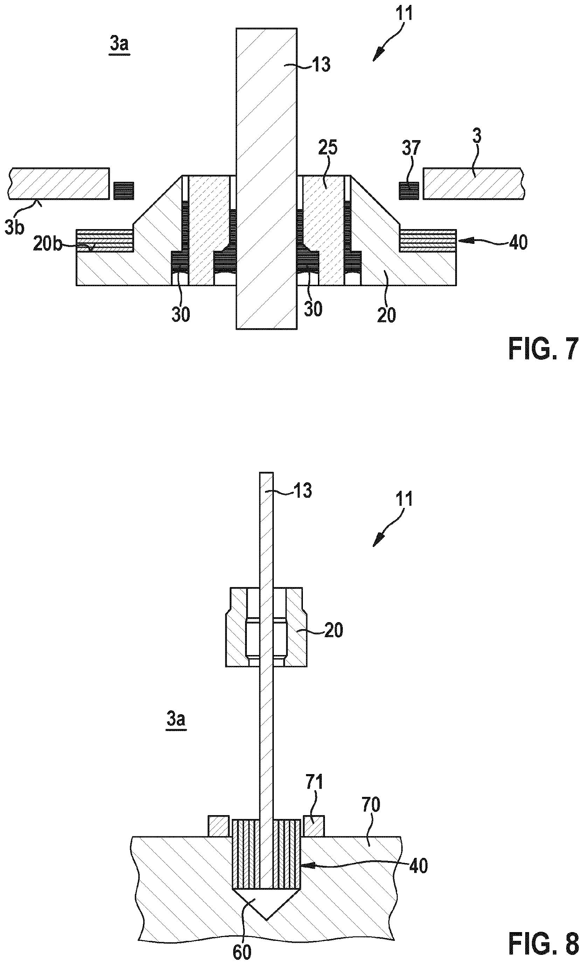

Der mindestens eine Stift 13 der elektrischen Durchführung 11 ist im Innenraum 3a des Gehäuses 3 insbesondere mit einem elektrischen Kontakt 71 bzw. einer Leiterbahn einer Leiterplatte 70 der elektronischen Schaltung 7 des Geräts 1 elektrisch leitfähig verbunden, insbesondere über eine Lötverbindung. In einer Ausführungsform der Erfindung wird diese Verbindung als Teil der elektrischen Durchführung betrachtet (vgl. Fig. 8).

Die Figuren 2 bis 8 zeigen einzelne Ausführungsformen des erfindungsgemäßen Verfahrens bzw. einer erfindungsgemäßen elektrischen Durchführung 11.

Bei dem Verfahren wird die Wärmeenergie zum Fügen zumindest einer ersten und einer zweiten Komponente der Durchführung 11 mit Hilfe eines reaktiven Multischichtsystems 40 an die Fügestelle gebracht. Hierbei können die beiden Komponenten mit Hilfe des reaktiven Multischichtsystems 40 durch Diffusion stoffschlüssig miteinander gefügt werden.

Insbesondere kann auf dem Multischichtsystem 40 ein Lotmaterial bereitgestellt werden, wobei das reaktive Multischichtsystem 40 das Lotmaterial aufschmilzt und somit eine stoffschlüssige Verbindung der beiden Komponenten über das Lotmaterial erzeugt.

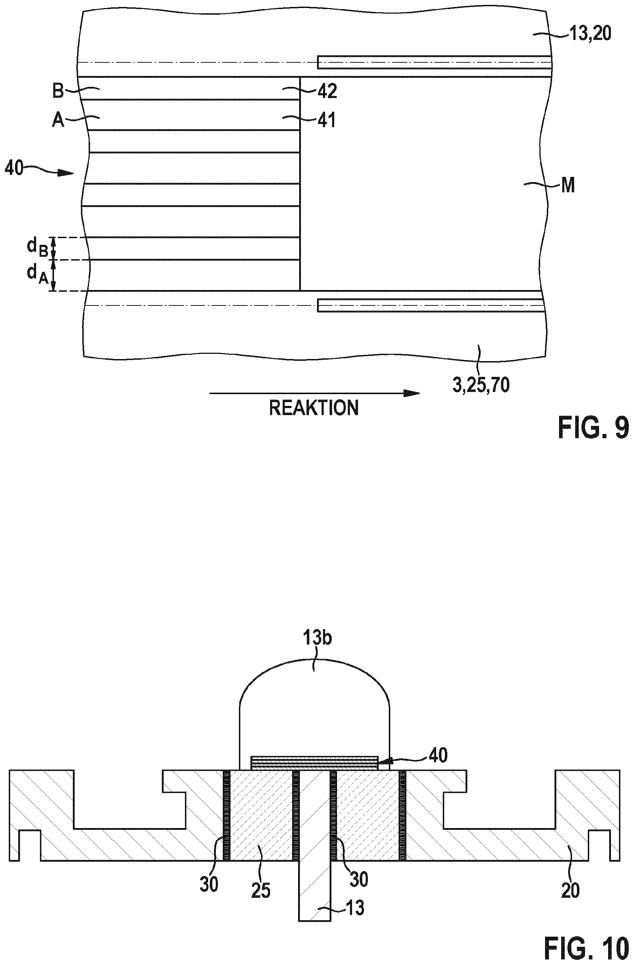

Ein erfindungsgemäß verwendbares reaktives Multischichtsystem 40 ist in der Figur 9 schematisch dargestellt. Das reaktive Multischichtsystem 40 besteht aus einer sich wiederholenden Schichtfolge einer ersten und einer zweiten Materiallage 41, 42 wobei die erste Materiallage einen ersten Stoff A aufweist und wobei die zweite Materiallage einen Stoff B aufweist. Die beiden Materiallagen 41, 42 weisen jeweils eine Schichtdicke d_{A} bzw. d_{B} auf, die im Nanometerbereich liegt (siehe z.B. oben). Das Multischichtsystem kann eine Gesamtzahl an Materiallagen aufweisen, die um Bereich von 2 bis 10000, vorzugsweise 10 bis 70, liegt (siehe z.B. oben).

Durch eine initiale Zündung, d.h., eine Anregung mit einer Anregungsenergie, reagieren die geschichteten Werkstoffe A, B des reaktiven Multischichtsystems 40 mit sich selbst exotherm und setzen hierbei Warme frei und bilden eine homogene Mischphase. Die freigesetzte Wärme wird dazu genutzt, Werkstoffe mittels Diffusion zu Bonden (siehe z.B. Tabelle oben). Hierbei kann die Mischphase selbst eine stoffschlüssige Verbindung zweier Komponenten der Durchführung ermöglichen bzw. bilden.

Um die betreffenden Komponenten bei niedriger Energie Löten zu können, kann das reaktive Multischichtsystem 40 dazu genutzt werden, ein eutektisches Lot zu schmelzen, welches auf dem reaktiven Multischichtsystem 40 aufgebracht wurde. Alternativ kann das Lot sehr nahe an dem reaktiven Multischichtsystem in einem Lotdepot platziert werden.

Das reaktive Multischichtsystem 40 kann grundsätzlich vorher auf zumindest eine der beiden zu fügenden Komponenten der Durchführung 11 aufgebracht werden. Zum Fügen wird die exotherme Reaktion gezündet (z.B. durch Druck, Laserlicht, Wärme, etc.). Die pro Mol freigesetzte Wärmemenge ist in der Regel eher gering, in Summe reicht die Wärme jedoch aus, um Lote zu schmelzen oder artgleiche Materialien (z.B. Titan und Titan) durch Diffusion zu Bonden.

Auf diese Weisen können Komponenten der Durchführung vormontiert werden, ohne dass eine Laserschweißung ausgeführt werden muss, ferner können alle Bauteile bzw. Komponenten bei der Endmontage in Endlage durch Zündung des reaktiven Multischichtsystems 40 verbunden werden.

Fig. 2 zeigt eine Ausführungsform eines erfindungsgemäßen Verfahrens bzw. einer erfindungsgemäßen elektrischen Durchführung 11, wobei das reaktive Multischichtsystem 40 zur stoffschlüssigen Verbindung des metallischen Flansches 20 mit einem hier nicht gezeigten metallischen Gehäuse 3 des Gerätes 1 dienen kann. Das Multischichtsystem 40 wird dabei vorzugsweise auf eine Oberfläche 20a des Flansches 20 der Durchführung 11 aufgebracht, die nach Herstellung der stoffschlüssigen Verbindung zwischen dem Flansch 20 und dem Gehäuse 3 dem Innenraum 3a des Gehäuses 3 des medizinischen Gerätes 1 abgewandt ist. Optional kann auf dem Multischichtsystem 40 ein Lotmaterial angeordnet sein, so dass auch eine Lötverbindung zum Gehäuse 3 möglich ist. Durch den Flansch 20 hindurch erstreckt sich ein elektrisch leitfähiger Stift 13 der Durchführung, der gegenüber dem Flansch 20 mit einem Isolator 25 isoliert ist, der einen Ringspalt zwischen dem Stift 13 und dem Flansch 20 ausfüllt. Der Isolator 25 kann aus einem Glas gebildet sein und dichtet den Ringspalt zwischen dem Stift 13 und dem Flansch 20 ab und stellt dabei eine stoffschlüssige Verbindung zwischen dem Stift 13 und dem Flansch 20 her.

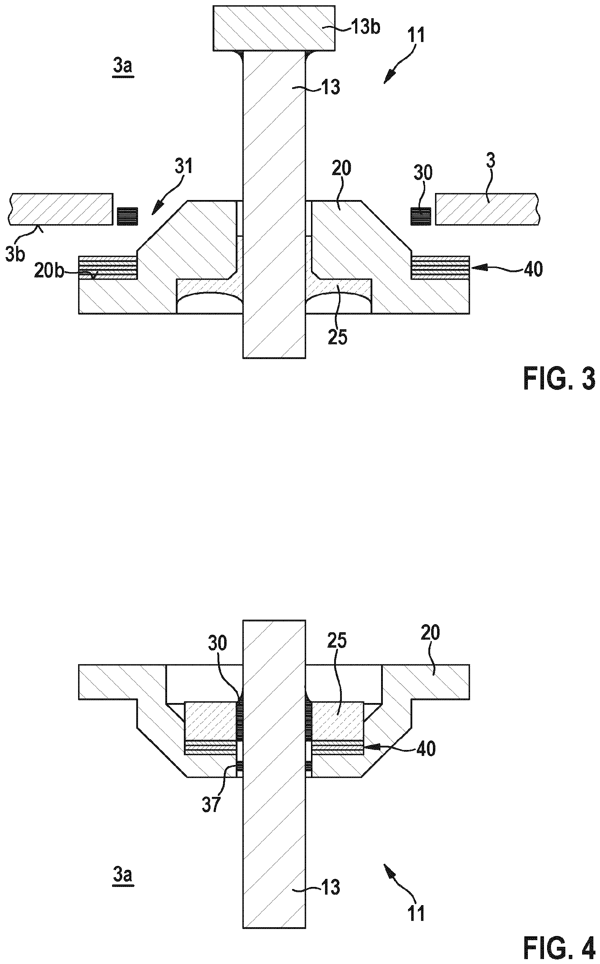

Fig. 3 zeigt eine weitere Ausführungsform eines erfindungsgemäßen Verfahrens bzw. einer erfindungsgemäßen elektrischen Durchführung 11, wobei ein metallischer Flansch 20 der Durchführung 11 und ein metallisches Gehäuse 3 des medizinischen Geräts 1 mittels eines reaktiven Multischichtsystems 40 und zusätzlichem Lotmaterial 30 zur Herstellung einer stoffschlüssigen Verbindung gefügt werden. Hierbei wird das Multischichtsystem 40 auf eine Oberfläche 20b des Flansches 20 aufgebracht, die nach Herstellung der stoffschlüssigen Verbindung zwischen Flansch 20 und Gehäuse 3 der Außenseite 3b des Gehäuses 3 zugewandt ist, d.h., die stoffschlüssige Verbindung wird zwischen der Außenseite 3b des Gehäuses 3 und der Oberfläche 20b des Flansches 20 gebildet. Hierzu wird zusätzlich ein Lotmaterial 30 auf dem Multischichtsystem 40 angeordnet, so dass es vorzugsweise in einem Spalt 31 zwischen dem Flansch 20 und dem Gehäuse 3 zu liegen kommt. Durch Anregen des Multischichtsystems 40 mit einer Aktivierungsenergie wird Wärme freigesetzt, die das Lotmaterial 30 aufschmilzt, so dass eine Lötverbindung zwischen dem Flansch 20 und dem Gehäuse 3 hergestellt wird.

Durch den Flansch 20 hindurch erstreckt sich wiederum ein elektrisch leitfähiger Stift 13 der Durchführung 11, der gegenüber dem Flansch 20 mit einem Isolator 25 isoliert ist, der in einem Ringspalt zwischen dem Stift 13 und dem Flansch 20 angeordnet ist. Der Isolator 25 kann aus einem Glas gebildet sein und dichtet den Ringspalt zwischen dem Stift 13 und dem Flansch 20 ab und stellt des Weiteren eine stoffschlüssige Verbindung zwischen dem Stift 13 und dem Flansch 20 her. Der Stift 13 kann im Innenraum 3a des Gehäuses 3 des medizinischen Geräts 1 ein lötbares Pad 13b aufweisen, z.B. zur Herstellung einer elektrisch leitfähigen Verbindung mit einer elektronischen Schaltung 7 des Geräts 1.

Fig. 4 zeigt eine weitere Ausführungsform eines erfindungsgemäßen Verfahrens bzw. einer erfindungsgemäßen elektrischen Durchführung 11, wobei hier ein metallischer Flansch 20 der Durchführung 11 und ein aus einer Keramik gebildeter Isolator 25 der Durchführung 11 mittels eines reaktiven Multischichtsystems 40 und optionalem Lotmaterial 37 gefügt werden. Das Multischichtsystem 40 wird hierzu zwischen dem Flansch 20 und dem Isolator 25 angeordnet, wobei vorzugsweise durch Zünden des Multischichtsystems 40 eine exotherme Reaktion der Stoffe A und B des Multischichtsystems (vgl. Fig. 9) gestartet wird, die in einer stoffschlüssigen Verbindung zwischen dem Flansch 20 und dem Isolator 25 resultiert. Optional kann ein Lotmaterial 37 in einem Ringspalt zwischen dem Flansch 20 und einem Stift 13 aufgeschmolzen werden (z.B. durch die exotherme Reaktion des Multischichtsystems 40), so dass eine Lötverbindung zwischen dem Stift 13 und dem Flansch 20 erzeugt wird. Der Stift 13 erstreckt sich durch den Isolator 25 hindurch und ist mit diesem über eine Lötverbindung 30 stoffschlüssig verbunden.

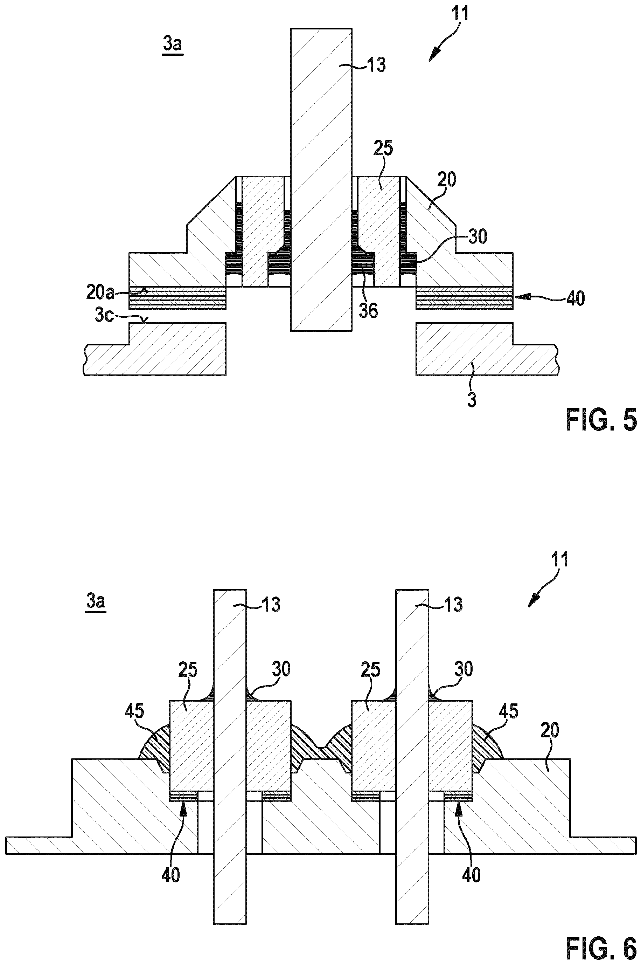

Fig. 5 zeigt eine weitere Ausführungsform eines erfindungsgemäßen Verfahrens bzw. einer erfindungsgemäßen elektrischen Durchführung 11, wobei ein metallischer Flansch 20 der Durchführung 11 und ein metallisches Gehäuse 3 des medizinischen Geräts 1 mittels eines reaktiven Multischichtsystems 40 gefügt werden.

Hierbei wird das Multischichtsystem 40 auf eine Oberfläche 20a des Flansches 20 aufgebracht, die nach Herstellung der stoffschlüssigen Verbindung zwischen Flansch 20 und Gehäuse 3 einer Innenseite 3c des Gehäuses 3 zugewandt ist, d.h., die stoffschlüssige Verbindung wird zwischen der Innenseite 3c des Gehäuses 3 und der Oberfläche 20a des Flansches 20 gebildet. Durch Anregen des Multischichtsystems 40 mit einer Aktivierungsenergie wird eine exotherme Reaktion der Stoffe A und B gestartet (vgl. Fig. 9) die eine stoffschlüssige Verbindung zwischen der Innenseite 3c des Gehäuses 3 und des Flansches 20 erzeugt. Optional kann auf dem Multischichtsystem 40 ein Lotmaterial, z.B. ein eutektisches Lotmaterial vorgesehen sein, so dass zwischen der Oberfläche 20a des Flansches 20 und der Innenseite 3c des Gehäuses 1 eine Lötverbindung erzeugt wird.

Der Flansch 20 umgibt weiterhin einen Isolator 25 aus einem keramischen Material und ist mit dem Isolator 25 über eine Lötverbindung 30 verbunden. Weiterhin erstreckt sich ein elektrisch leitfähiger Stift 13 der Durchführung 11 durch den Isolator 25 hindurch und ist mit dem Isolator 25 ebenfalls über eine Lötverbindung 36 verbunden, so dass sich eine hermetisch abgedichtete Baugruppe aufweisend den Flansch 20, den Isolator 25 und Stift 13 ergibt.

Fig. 6 zeigt eine weitere Ausführungsform eines erfindungsgemäßen Verfahrens bzw. einer erfindungsgemäßen elektrischen Durchführung, wobei ein metallischer Flansch 20 der Durchführung 11 und zwei Isolatoren 25 der Durchführung 11, die aus einem keramischen Material gebildet sind, jeweils mittels eines reaktiven Multischichtsystems 40 gefügt werden, wobei eine zusätzliche Kunststoffdichtung 45 zum Abdichten der Isolatoren 25 verwendet wird.

Das Multischichtsystem 40 wird hierzu zwischen dem Flansch 20 und dem jeweiligen Isolator 25 angeordnet, wobei vorzugsweise durch Zünden des Multischichtsystems 40 eine exotherme Reaktion der Stoffe A und B des Multischichtsystems (vgl. Fig. 9) gestartet wird, die in einer stoffschlüssigen Verbindung zwischen dem Flansch 20 und dem jeweiligen Isolator 25 resultiert. Durch den jeweiligen Isolator 25 erstreckt sich weiterhin ein Stift 13 hindurch, der mit dem betreffenden Isolator 25 über eine Lötverbindung 30 stoffschlüssig verbunden ist. Zusätzlich ist vorzugsweise ein Kunststoffdichtung 45 vorgesehen, die den Flansch 20 mit den beiden Isolatoren 25 verbindet um eine zusätzliche Abdichtung der Verbindungen zwischen dem Flansch 20 und den Isolatoren 25 bereitzustellen.

Fig. 7 zeigt eine weitere Ausführungsform eines erfindungsgemäßen Verfahrens bzw. einer erfindungsgemäßen elektrischen Durchführung 11, wobei ein metallischer Flansch 20 der Durchführung 11 und ein metallisches Gehäuse 3 des medizinischen Geräts 1 mittels eines reaktiven Multischichtsystems 40 und ggf. optionalem Lotmaterial 37 zur Herstellung einer stoffschlüssigen Verbindung gefügt werden. Hierbei wird das Multischichtsystem 40 auf eine Oberfläche 20b des Flansches 20 aufgebracht, die nach Herstellung der stoffschlüssigen Verbindung zwischen Flansch 20 und Gehäuse 3 der Außenseite 3b des Gehäuses 3 zugewandt ist, d.h., die stoffschlüssige Verbindung wird zwischen der Außenseite 3b des Gehäuses 3 und der Oberfläche 20b des Flansches 20 gebildet. Hierbei kann zusätzlich ein Lotmaterial 30 auf dem Multischichtsystem 40 angeordnet werden, so dass es z.B. in einem Spalt 31 zwischen dem Flansch 20 und dem Gehäuse 3 zu liegen kommt. Durch Anregen des Multischichtsystems 40 mit einer Aktivierungsenergie wird eine exotherme Reaktion der Stoffe A und B gestartet (vgl. Fig. 9) die eine stoffschlüssige Verbindung zwischen der Außenseite 3b des Gehäuses 3 und der Oberfläche 20b des Flansches 20 erzeugt. Ist zusätzlich Lotmaterial 37 vorgesehen, wird vermittels der Reaktionswärme eine Lötverbindung zwischen dem Flansch 20 und dem Gehäuse 3 hergestellt.

Durch den Flansch 20 hindurch erstreckt sich wiederum ein elektrisch leitfähiger Stift 13 der Durchführung 11, der gegenüber dem Flansch 20 mit einem Isolator 25 isoliert ist, der in einem Ringspalt zwischen dem Stift 13 und dem Flansch 20 angeordnet ist. Der Isolator 25 ist z.B. aus einem keramischen Material gebildet und kann mit dem Flansch 20 einerseits und dem Stift 13 andererseits jeweils über eine Lötverbindung 30 verbunden sein.

Fig. 8 zeigt eine weitere Ausführungsform eines erfindungsgemäßen Verfahrens bzw. einer erfindungsgemäßen elektrischen Durchführung, wobei hier ein Stift 13 der Durchführung 11 und ein Kontakt 71 einer Leiterplatte 70 des medizinischen Geräts 1 mittels eines reaktiven Multischichtsystems 40 gefügt werden. Die Leiterplatte 70 kann z.B. die elektronische Schaltung 7 tragen (vgl. Fig. 1). Insbesondere kann ein Endabschnitt des Stiftes 13, auf dem das reaktive Multischichtsystem 40 angeordnet ist, in einer Ausnehmung 60 der Leiterplatte 70 angeordnet werden. Bei der Ausnehmung 60 kann es sich z.B. um eine Bohrung oder ein VIA handeln. Durch Anregen des Multischichtsystems 40 mit einer Aktivierungsenergie wird eine exotherme Reaktion der Stoffe A und B gestartet (vgl. Fig. 9) die eine stoffschlüssige, elektrisch leitfähige Verbindung zwischen dem Stift 13 und dem Kontakt 71 der Leiterplatte 70 erzeugt. Optional kann auch hier auf dem Multischichtsystem 40 ein Lotmaterial vorgesehen werden, so dass das Lotmaterial nach dem Aufschmelzen und Erstarren eine elektrische Verbindung zu dem Kontakt 71 herstellt.

In den oben beschriebenen Ausführungsformen gemäß Figuren 2 bis 8 kann das jeweilige reaktive Multischichtsystem 40 z.B. gemäß Figur 9 ausgebildet sein. Das Multischichtsystem 40 kann dabei die oben beschriebenen Stoffkombinationen der Stoffe A und B sowie Dimensionen aufweisen (Gesamtanzahl der Materiallagen 41, 42 und Schichtdicken d_{A}, d_{B} der Materiallagen 41, 42). Weiterhin können der Flansch 20 sowie das Gehäuse 3 jeweils aus einer der oben beschriebenen Metalllegierungen gebildet sein. Der Stift 13 kann jeweils die oben genannten Metalle aufweisen.

Das erfindungsgemäße Verfahren erweist sich als vorteilhaft, da die Wärmebelastung für das medizinische Gerät 1 minimal ist; denn das Gehäuse 3 bzw. Gerät 1 muss nicht durchwärmt werden, sondern die benötigte Fügeenergie kann lokal in der Fügezone erzeugt werden. Aufgrund der Erfindung können problembehaftete Laserschweißungen entfallen.

Die Zündung des reaktiven Multischichtsystems kann auf verschiedenen Wegen erfolgen. Zum Beispiel mittels eines Laserimpulses. Ist die Aktivierungsenergie zum Zünden der Reaktion ausreichend, so breitet sich die Reaktion im reaktiven Multischichtsystem von alleine aus. Eine Prozesskontrolle bzw. Steuerung ist daher nicht erforderlich.

Weiterhin ist eine erfindungsgemäße Fügung auch an schwer zugänglichen Stellen ohne direkten Sichtkontakt möglich und kann z.B. mittels Druck ausgelöst werden. Dies ist insbesondere zur Herstellung von sehr kleinen oder schwer herstellbaren Gehäusekomponenten hilfreich, welche nicht mittels eines Laserstrahls gefügt werden können, da die Gefahr besteht, dass der Laserstrahl während des Einschweißens das Bauteil zu stark erhitzt (Überhitzung) oder das Bauteil durch die Strahlung schädigt (durchschweißt).

Fig. 10 zeigt eine weitere Ausführungsform eines erfindungsgemäßen Verfahrens bzw. einer erfindungsgemäßen elektrischen Durchführung, wobei hier der Stift 13 der Durchführung 11 mittels eines Kontaktelements 13a verlängert wurde. Das Kontaktelement 13a kann aus den beschriebenen Werkstoffen eines Stiftes hergestellt werden. Die mechanische Fügeverbindung wird mittels des reaktiven Multischichtsystems 40 an dem bereits vorhandenen Stift 13 und/oder an dem Isolator 25 angefügt. Um die Fügeverbindung zusätzlich zu verbessern kann eine weitere Lötverbindung neben dem reaktiven Multischichtsystem 40 eingesetzt werden. Die elektrische Verbindung durch beiden Elemente des Stiftes 13a und 13 wird ebenfalls durch das reaktive Multischichtsystem 40 hergestellt.

Wird das Kontaktelement 13a auf der körperzugewandten Seite eigesetzt, ist es vorteilhaft den Abschluss aus biokompatiblen Metallen, wie z.B. Titan, Tantal, Platin, Iridium o.ä. herzustellen. Wird das Kontaktelement 13a auf der körperabgewandten Seite der Durchführung eingesetzt, so ist es vorteilhaft es aus weichlötbaren oder schweißbaren Metallen, wie z.B. Kupfer, Nickel, Eisen zu fertigen.

## Patentansprüche

1. Verfahren zum Fügen einer ersten (20, 13) und einer zweiten Komponente (3, 25, 70) einer elektrischen Durchführung (11) für ein medizinisches Gerät (1), aufweisend die Schritte:
- Bereitstellen der ersten Komponente (20, 13), der zweiten Komponente (3, 25, 70) und eines reaktiven Multischichtsystems (40) aufweisend alternierend angeordnete erste und zweite Materiallagen (41, 42), wobei die ersten Materiallagen (41) einen ersten Stoff (A) aufweisen und wobei die zweiten Materiallagen (42) einen zweiten Stoff (B) aufweisen, und
- Anregen des reaktiven Multischichtsystems (40) mit einer Aktivierungsenergie, so dass die beiden Stoffe (A, B) unter Freisetzung von Wärmeenergie exotherm miteinander reagieren, wobei die Wärmeenergie zum Fügen der ersten und der zweiten Komponente (20, 13; 3, 25, 70) verwendet wird.

2. Verfahren nach Anspruch 1, **wobei** eine stoffschlüssige Verbindung zwischen der ersten und der zweiten Komponente (20, 13; 3, 25, 70) durch ein Mischphase (M) des ersten und des zweiten Stoffes (A, B) gebildet wird.

3. Verfahren nach Anspruch 1 oder 2, **wobei** das reaktive Multischichtsystem (40) vor dem Anregen zwischen der ersten und der zweiten Komponente (20, 13; 3, 25, 70) angeordnet wird.

4. Verfahren nach einem der vorhergehenden Ansprüche, **wobei** auf dem reaktiven Multischichtsystem (40) weiterhin ein Lotmaterial (30, 37) angeordnet ist, insbesondere ein eutektisches Lotmaterial, wobei das Lotmaterial (30, 37) durch die Wärmenergie aufgeschmolzen wird, so dass eine Lötverbindung zwischen der ersten und der zweiten Komponente (20; 3) ausgebildet wird.

5. Verfahren nach einem der vorhergehenden Ansprüche, **wobei** die Aktivierungsenergie zur Anregung des reaktiven Multischichtsystems (40) durch Bestrahlen des Multischichtsystems (40) mit Laserlicht bereitgestellt wird.

6. Verfahren nach einem der vorhergehenden Ansprüche, **wobei** die Aktivierungsenergie zur Anregung des reaktiven Multischichtsystems (40) durch Ausüben eines mechanischen Drucks auf das reaktive Multischichtsystem (40) bereitgestellt wird.

7. Verfahren nach einem der vorhergehenden Ansprüche, **wobei** die erste Komponente ein metallischer Flansch (20) der elektrischen Durchführung (11) ist.

8. Verfahren nach einem der vorhergehenden Ansprüche, **wobei** die zweite Komponente ein metallisches Gehäuse (3) des medizinischen Gerätes (1) ist.

9. Verfahren nach einem der Ansprüche 1 bis 7, **wobei** die zweite Komponente ein Isolator (25) der elektrischen Durchführung (11) ist, wobei sich zumindest ein elektrisch leitfähiger Stift (13) der elektrischen Durchführung (11) durch den Isolator (25) hindurch erstreckt.

10. Verfahren nach einem der Ansprüche 1 bis 6, **wobei** die erste Komponente ein elektrisch leitfähiger Stift (13) der elektrischen Durchführung (11) ist, der sich insbesondere durch einen Isolator (25) der elektrischen Durchführung (11) hindurch erstreckt.

11. Verfahren nach einem der Ansprüche 1 bis 6 oder nach Anspruch 10, **wobei** die zweite Komponente eine Leiterplatte (70) einer elektrischen Schaltung (7) ist.

12. Verfahren nach einem der vorhergehenden Ansprüche, **wobei** der erste Stoff (A) und der zweite Stoff (B) aus der Gruppe der folgenden Kombinationen ausgewählt sind, wobei jeweils zunächst der erste Stoff (A) und dann der zweite Stoff (B) genannt ist:
- Al und Ni,
- Al und Pt,
- Al und Zi,
- Nb und Si,
- Ni und Ti,
- Si und Ti,
- Pt und Al,
- Ti und Si,
- Ti und C.

13. Verfahren nach einem der vorhergehenden Ansprüche, **wobei** das reaktive Multischichtsystem (40) eine Gesamtanzahl von Materiallagen (41, 42) aufweist, die im Bereich von 2 Materiallagen bis 10000 Materiallagen liegt, vorzugsweise im Bereich von 10 Materiallagen bis 70 Materiallagen.

14. Verfahren nach einem der vorhergehenden Ansprüche, **wobei** die jeweilige erste Materiallage (41) eine Schichtdicke (d_{A}) im Bereich von 1 nm bis 2000 nm aufweist, vorzugsweise im Bereich von 20 nm bis 500 nm, und/oder wobei die jeweilige zweite Materiallage (42) eine Schichtdicke (d_{B}) im Bereich von 1 nm bis 2000 nm aufweist, vorzugsweise im Bereich von 20 nm bis 500 nm.

15. Elektrische Durchführung (11) für ein medizinisches Gerät (1), insbesondere für ein implantierbares medizinisches Gerät (1), wobei die elektrische Durchführung (11) eine erste und eine zweite Komponente (20, 13; 3, 25, 70) aufweist, die mittels des Verfahrens nach einem der vorhergehenden Ansprüche gefügt sind.
